# EUROPEAN PATENT SPECIFICATION

(11) **EP 3 600 200 B1**
(45) Date of publication and mention of the grant of the patent: **10.12.2025**
(21) Application number: 17737074.9
(22) Date of filing: 23.03.2017
(51) Int. Cl.: A61F 13/514

(54) **ABSORBENT ARTICLE COMPRISING ELASTOMERIC STRIPS**
SAUGFÄHIGER ARTIKEL MIT ELASTOMEREN STREIFEN
ARTICLE ABSORBANT COMPRENANT DES BANDES ÉLASTOMÈRES

(43) Date of publication of application: 05.02.2020
(73) Proprietor: Hayat Kimya San. A.S., Uskudar, 34662 Istanbul (TR)
(72) Inventor: KOC, Fikret, 41275 Basiskele/Kocaeli (TR); KARATEKIN, Arzu, 41275 Basiskele/Kocaeli (TR); CANBOLAT, Eylem, 41275 Basiskele/Kocaeli (TR); ACAR, Haluk Ozgür, 41275 Basiskele/Kocaeli (TR); AKAYTAY, Arif, 41275 Basiskele/Kocaeli (TR)
(74) Representative: Dericioglu, E. Korhan
(86) International application number: PCT/TR2017/050112
(87) International publication number: WO 2018/174834

(56) References cited:
- WO-A1-01/28480
- US-A- 5 259 902
- US-A1- 2003 069 555

## Description

### FIELD OF THE INVENTION

The present invention relates to disposable or wearable absorbent articles such as; but not limited to, baby diapers, pants, pull up diapers, adult incontinence articles, which provide fit shape structure and antisagging property to absorbent articles by attaching at least one elastomeric strand and/or strip on/in the backsheet.

### BACKGROUND OF THE INVENTION

Absorbent articles for personal hygiene such as; disposable or wearable absorbent articles are designed to absorb or retain body exudates, in particular urine or feces. These kinds of absorbent articles can be baby diapers, training pants, pull up diapers or adult incontinence products. These absorbent articles have multiple layers having different functions. Basically the absorbent article is known to comprise a chasis being formed by a topsheet, a backsheet and an absorbent core layered between said topsheet and backsheet. It is also known to use acquisiton and/or distribution layer (ADL) between the topsheet and absorbent core structure.

The layers of the absorbent article can be produced by using various kinds of polymers according to their specified features.

The topsheet is layered on the top of the absorbent article that allows urine to pass through absorbent core layer. The topsheet is layered on the interior part of the absorbent article which contacts directly with the skin of the wearer. Topsheet materials are mostly chosen from softer nonwoven (NW) materials, as the topsheet is directly in touch with the skin of the wearer.

The acquisiton distribution layer (ADL) is functionilized to distribute the taken liquid or feces from topsheet layer and spread it on absorbent core proportionally. The acquisiton distribution layer is generally formed of spun bonded NW material to transfer the liquid taken from topsheet to the absorbent core.

The absorbent core structure is designed to absorb the urine and fluid feces and retains it. The absorbent core structure generally requires superabsorbent polymer material and/or cellulose material. Due to the absorbent materials used in the core region, large quantity of urine can be absorbed.

The absorbent core may be covered with core wrap made up of a nonwoven web structure.

The backsheet is layered as the bottom layer of the absorbent article to protect the body and the garments from the body waste. It forms the exterior surface of the absorbent article and prevents the leakage of the liquid or feces from the absorbent structure.

The design of the disposable or wearable absorbent article is shaped to fit the body structure while being worn. The form of the crotch area is shaped by taking the body shape into consideration.

Absorbent articles are desired to be flexible in the crotch region which will provide free movement of user. The benefits of the flexible crotch region are; improved freedom of movement for the wearer especially when the user's legs compress the crotch region of the absorbent article and/or improved performance of absorbent structure during usage. Despite the fact that, highly flexible products may in generally have a poor resiliency when becoming wet and thus tend to lose their shape while in use. Since the crotch region is compressed by the movement of the wearer's legs especially babies and the shape of the absorbent article is deformed. As the absorbent core is deformed, the product may fail performing properly which increases the likelihood of failure such as fluid leakages.

The core structure is designed to absorb the urine and/or feces and retains them. By having heavy feces and urine content, absorbent core becomes weighty and the absorbent article stretches downward while being used. Consequently, the absorbent article loses its shape by compressing the crotch portion with the wearer's legs which usually causes sagging which is a problem for the wearer, especially for the babies who are eager to play and trying to walk.

The main problem to be solved in the present invention is sagging of the absorbent articles when in use.

Forming channels on crotch region to provide fit shape structure to the wearer to overcome sagging problem is known in the art. These channels are generally formed by shaping absorbent core layer with the help of adhesives. These kinds of absorbent articles have absorbent cores covered by a core wrap enclosing absorbent material wherein at least 80% of the absorbent material is superabsorbent polymers. Generally the absorbent core does not contain any cellulosic material. The shape of these kinds of absorbent articles require a new machine application designed to be used in industrial production which is costly.

US2014163503 describes a disposable absorbent article comprising at least two channel forming areas formed by attaching the upper and bottom sides of an absorbent core wrap together with the help of an adhesive. Channel forming areas are free of absorbent particle and the absorbent core is free of cellulose fibers. When the article becomes wet the channels are formed in the crotch region. The absorbent article seems to be a traditional article when it is dry, and when the article is filled the channels are formed. US5259902 describes further prior art.

The swelled channels are not robust therefore not permenant. When the user sits down on his/her buttocks roughly, the channels can be deformed by bursting the channels from the adhesive applied parts.

Using only superabsorbent polymers in absorbent core sheet may cause a softness problem for users, especially for babies. There is a need to use cellulosic fibers to obtain softer and fluffy products.

Moreover, there is a need to use a different machine application in the industry to obtain that kind of absorbent article having channels which are free of superabsorbent polymers. With traditional diaper manufacturing equipments it is not possible to obtain that kind of structure, which is in industrial applications not economically feasable.

Additionally, in these kinds of applications, the absorbent core structure contains mostly superabsorbent polymers which will not be feasable again to prevent leakage. Large amounts of superabsorbent polymers is to be used to absorb large amounts of liquids, but using such large amounts is not economically feasable.

In the view of the above mentioned aspects there is a need to provide disposable absorbent articles which do not display above mentioned swollen shape and sagging problem. There is a need to provide fit shape structure of absorbent articles in filled state with an economical, feasable and simple manner.

### SUMMARY OF THE INVENTION

The present invention provides a pant or diaper as claimed in claim 1.

The present invention describes an absorbent article such as disposable or wearable absorbent article comprising; a chassis including a topsheet, a backsheet, an absorbent core located between said topsheet and backsheet and a liquid acquisition distribution layer located preferably between said topsheet and absorbent core.

The backsheet of the absorbent article has an interior face comprising a polymeric film layer; that is oriented as body facing side toward the wearer. The backsheet of the absorbent article has an exterior face comprising NW web layer; which is directly in contact with the garment of the wearer, when the absorbent article is being worn.

The backsheet comprises at least one strip comprising at least one strand, attached in/on the backsheet. The backsheet comprises at least one strip comprising at least one strand, attached in/on the polymeric film layer.

The strip(s) is formed by one or more strand(s), coming together to form channel like structure.

The elastomeric strand(s) is stretched to extend longitudinally before attaching on the backsheet. The extended elastomeric strand(s) and/or strip(s) is attached preferably on the surface of polymeric film layer of the backsheet by applying adhesives. The elastomeric strand(s) and/or strip(s) behaves like channel when the diaper is wet in use and holds the diaper up, to prevent sagging. The elastomeric strand(s) and/or strip(s) provides a fit shape structure to absorbent article when in use and also not in use.

### BRIEF DESCRIPTION OF DRAWINGS

**Fig. 1****.** Plan view of the layers of the absorbent article in flat position, as described in the present invention
**Fig. 2****.** Plan view of the backsheet showing the layers as, polymeric film layer and nonwoven web layer according to the present invention.
**Fig. 3****.** Top view of the disposable diaper in flat position, having single elastomeric strand(s) attached on backsheet as described in the present invention
**Fig. 4****.** Top view of the disposable diaper in flat position, having double elastomeric strand(s) in strip attached on backsheet as described in the present invention
**Fig. 5****.** Top view of the disposable diaper in flat position, having triple elastomeric strand(s) in strip attached on backsheet as described in the present invention
**Fig. 6****.** Front view of the wearable pant when in use having single elastomeric strand(s) attached on backsheet as described in the present invention
**Fig. 7****.** Front view of the wearable pant when in use having double elastomeric strand(s) in strip attached on backsheet as described in the present invention
**Fig. 8****.** Front view of the wearable pant when in use having triple elastomeric strands in strip attached on backsheet as described in the present invention.
**Fig.9****.** Top view of the disposable diaper in flat position, having double elastomeric strands in strip attached on backsheet;
   **9a.** in stretched state as described in the present invention
   **9b.** in relaxed state as described in the present invention

Wherein;
1: absorbent article
2: diaper
4: pant
6: topsheet
8: acquisition and/or distribution layer (ADL)
10: absorbent core
12: backsheet
14: elastomeric strand
16: elastomeric strip
18: polymeric film layer
20: nonwoven web layer
X: longitudinal axis
Y: lateral axis
A: the distance between two strands
B: the distance between two strips
W: width

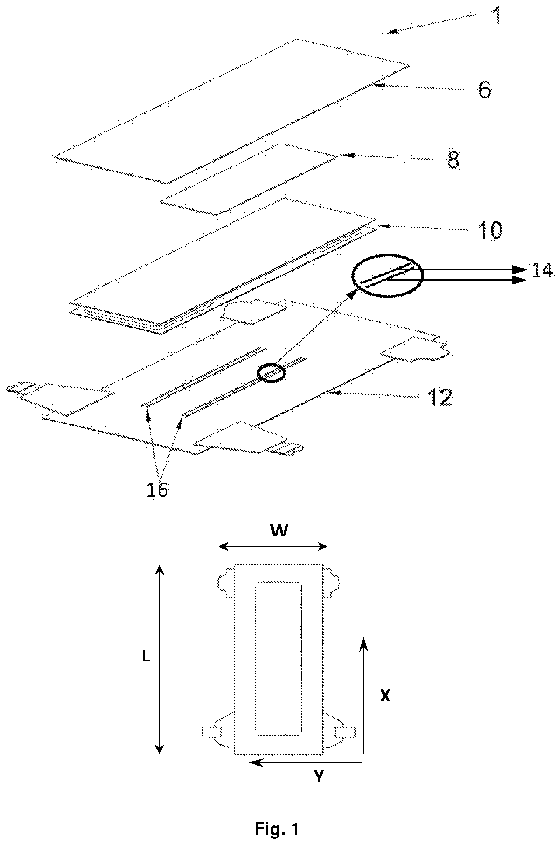

Fig. 1 is a plan view of the exemplary diaper, in flattened state, with elastomeric strips (16) which are formed by a plurality of elastomeric strands (14) on backsheet layer of the absorbent article.

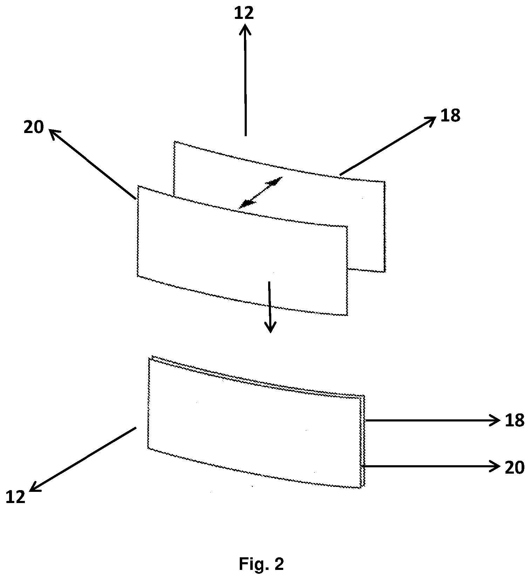

Fig. 2 is a plan plan view of the backsheet comprising two layers. The interior layer of the backsheet is polymeric film layer and the exterior layer of the backsheet is nonwoven web structure.

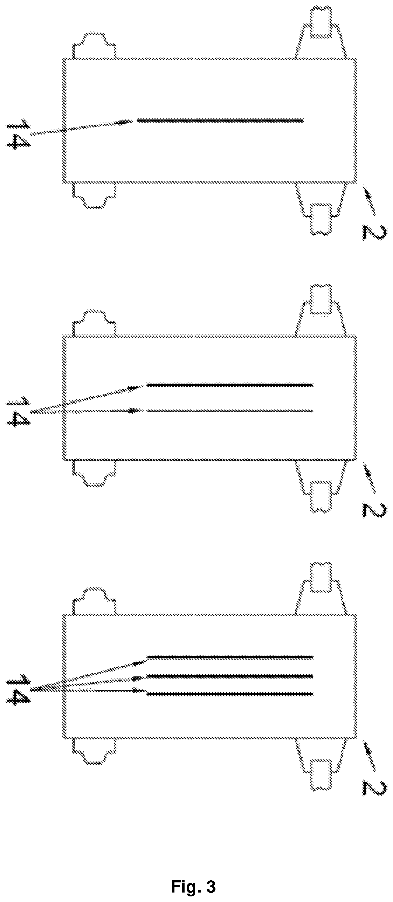

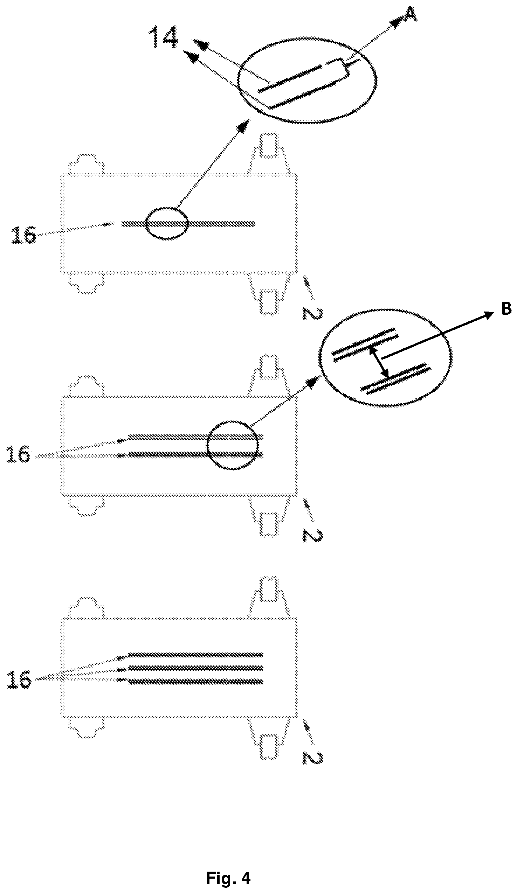

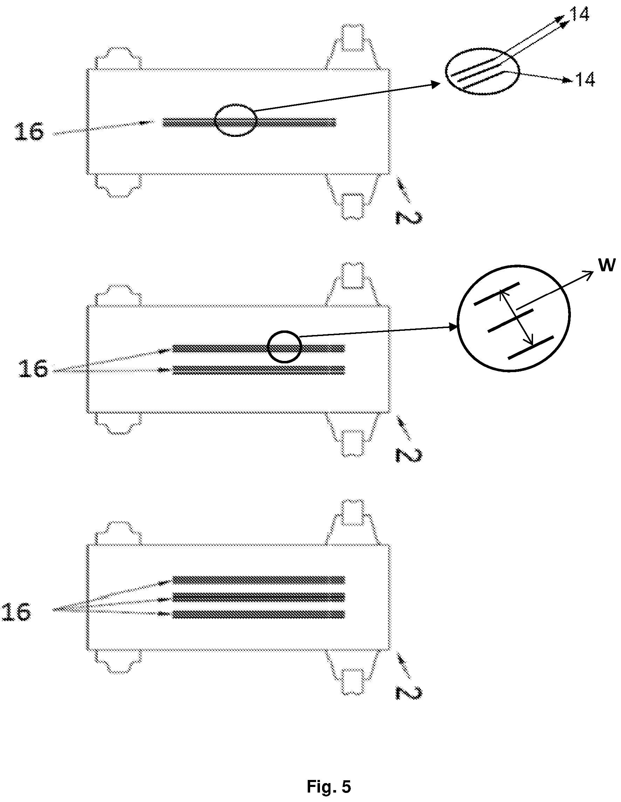

Exemplary absorbent articles according to the present invention in the form of a diaper is represented in Figs. 3-5.

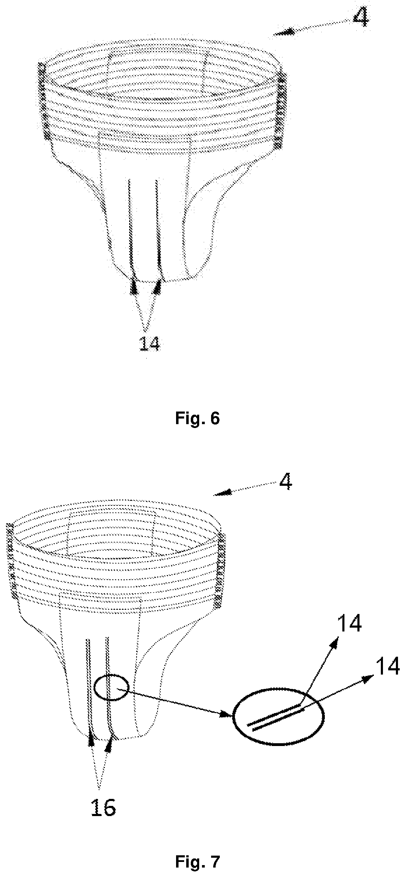

Exemplary absorbent articles according to the present invention in the form of a pant or pull up is represented in Figs. 6-8.

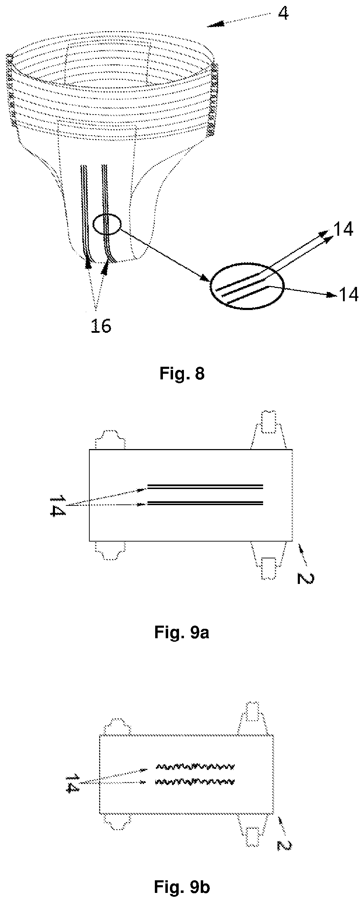

Fig. 9a is a plan view of the exemplary diaper, in flattened state with double elastomeric strips (16) comprising double elastomeric strands (14) in each, attached on backsheet wherein, the absorbent article is in stretched state and the elastomeric strands are lying in straight shape.

Fig. 9b is a plan view of the exemplary diaper, in flattened state with double elastomeric strips (16) comprising single elastomeric strands (14) in each, attached on backsheet wherein, the absorbent article is in relaxed state and the elastomeric strands are in crinkled shape.

The shown diaper and/or pant structures are shown only for illustration purpose, as the present invention may be applied for making a wide variety of absorbent articles.

### ABBREVIATIONS

NW: Nonwoven
ADL: Acquisition and/or distrubition layer
SAP: Super absorbent polymer
L: Length
W: Width

### DESCRIPTION OF THE INVENTION

The present invention is related to a pant or diaper (1) such as, but not limited to, baby diapers, training pants and adult incontinence products comprising elastomeric strand(s) (14) and/or strip(s) (16) to provide fit shape structure and antisagging property to absorbent article (1).

The term absorbent article used herein refers to disposable or wearable absorbent articles such as baby diapers (2), training pants (4), adult incontinence products, and the like which are put on to absorb bodily fluids and retain the various exudates discharged from the body. Typically these articles comprise a topsheet (6), a backsheet (12), an absorbent core (10) and optionally an acquisition/distrubition (8) layer which may be comprised of one or more layers and typically other components, such as fastening members, cuffs and frontal tape structure.

The present invention relates to a pant or diaper (1) providing an improved fit in both dry and wet states and providing improved comfort for the wearers.

Dry state refers to, the absorbent article (1) being worn to the wearer but not loaded with body liquid or feces, or to the absorbent article (1) not being worn yet.

Wet state refers to, the absorbent article (1) which is in use and loaded with bodily fluids or feces.

The present invention relates to an absorbent article (1) having antisagging property in loaded and not loaded state during the use.

The pant or diaper (1) in the present invention comprises a liquid permeable topsheet (6), a liquid impermeable backsheet (12), an absorbent core (10) located between the topsheet (6) and the backsheet (12), and an acquisition-distribution layer (ADL) (8), located between the topsheet (6) and the absorbent core (10). The absorbent article (1) may also comprise a liquid impermeable barrier leg cuffs substantially planar with the chassis of the absorbent article (1).

The present invention is disclosed with the given non-limiting embodiments;

In one embodiment, the backsheet (12) of the absorbent article (1) is shaped with at least one strand (14) and/or strip (16), as like having channels when the absorbent article (1) is in dry and in wet state during the use.

According to the present application, formed channels can not be deformed by external forces, because the channels are formed permenantly with the help of hot melt adhesive used for attaching the elastomeric strand(s) (14) and/or strip(s) (16)-on the backsheet (12).

Additionally, as an advantage of the present invention, superabsorbent polymers and/or cellulose fibers can be used as absorbent core (10) material separately or in mixtures.

In one embodiment, said elastomeric strand(s) (14) and/or strip(s) (16) is extended longitudinaly up to some extend before attaching on the backsheet (12) in flat position.

In one embodiment, used elastomeric strand(s) (14) and/or strip(s) (16); is attached on at least one face of the backsheet (12) with help of adhesives.

In one embodiment, said elastomeric strand(s) (14) and/or strip(s) (16) is extended longitudinaly up to some extend before attaching in/on the polymeric film layer (18) of the backsheet (12) in flat position.

In one embodiment, said elastomeric strand(s) (14) and/or strip(s) (16) is extended longitudinaly up to some extend before attaching on the polymeric film layer (18) of the backsheet (12) in flat position.

In one embodiment, said elastomeric strand(s) (14) and/or strip(s) (16) is extended longitudinaly up to some extend before attaching on the interior surface of the polymeric film layer (18) of the backsheet (12) that is in contact with the absorbent core (10).

In one embodiment, said elastomeric strand(s) (14) and/or strip(s) (16) is extended longitudinaly up to some extend before attaching on the exterior surface of the polymeric film layer (18) of the backsheet (12) that is in contact with the nonwoven web layer (20).

In one embodiment, said elastomeric strand(s) (14) and/or strip(s) (16) is extended longitudinaly up to some extend before attaching in the polymeric film layer (18) of the backsheet (12) in flat position.

In the present invention; the term of elastomeric strand used herein means, only one string or band formed by elastomeric polymer material. And the term of elastomeric strip used herein means that, one elastomeric strand (14) has a width up to some extend or more than one elastomeric strands (14) come together as plurality of elastomeric strands (14) to form an elastomeric strip (16). At least one elastomeric strand (14) having a width up to some extend may form an elastomeric strip (16).

According to the present invention;

Topsheet (6), is placed on the top of the absorbent article (1) as the interior side of the article, that can be formed of spun bonded or melt blown nonwoven structure.

In the present invention, topsheet (6) is formed of a liquid permeable spunbonded nonwoven web structure (20). The function of said topsheet (6) is taking the body exudates through the bottom layers.

In the present invention, the acquisition distribution layer (8) (ADL) is positioned between the topsheet (6) and the absorbent core (10). The function of the acquisition distribution layer (8) is taking the body fluid and distributing it through the absorbent core (10) to spread it proportionally. In the present invention the ADL (8) is formed from spun bonded NW web structure.

In the present invention, the absorbent core (10) is placed between the backsheet (12) and the acquisition distribution layer (8) or topsheet (6). The function of said absorbent core (10) is to absorb and retain the exudates of the body for a prolonged amount of time. For example; to minimize re-wet to keep the wearer dry and avoid soiling of clothes or bed sheets overnight.

In the present invention, absorbent article (1) comprises any known absorbent material such as; a blend of wood pulp such as, cellulose with superabsorbent polymers (SAP) in particulate form, also called absorbent gelling materials. The absorbent core (10) layer may comprise both of them or the absorbent core may only comprise SAP as absorbent materials to have a thinner nature in unloaded state.

In the present invention, the absorbent article (1) has an absorbent core (10) comprising an absorbent material such as; SAP and/or cellulose.

In the present invention the backsheet (12) is located under the absorbent core (10). Said backsheet (12) of the present invention is formed of any known conventional structure that, comprising at least two layers. One of these two layers is formed of nonwoven (NW) web structure (20) and the other one is formed of a polymeric film layer (18).

In the present invention, the backsheet may comprise more than one layer of polymeric film layer (18).

In the present invention, the backsheet may comprise more than one layer of nonwoven web layer (20)
The backsheet (12) of the present invention is of liquid impervious nature. The function of said backsheet (12) is, to prevent body and clothes of the wearer from exudates by retaining the liquid or feces inside the absorbent article (1).

Nonwoven web layer used herein may be a manufactured web sheet, formed by directionally or randomly orientated fibers, bonded by spun bonding or melt blown bonding methods. The fibers may be of natural or man-made nature and may be staple or continuous filaments. Commercially available fibers can be used as NW web fiber. The fibers come in several different forms such as short fibers as; staple, or chopped; continuous single fibers as filaments or monofilaments.

Nonwoven web layer (20) of the present invention can be formed by any known process such as meltblowing, spunbonding, solvent spinning, electrospinning, carding and airlaying. The basis weight of nonwoven webs used in the present invention differs for topsheet (6), ADL (8) or backsheet (12).

Polymeric film layer (18) used in the disclosure means; a film layer of a polymeric material. Polymeric film (18) used in backsheet (12) of the absorbent article (1) can be any known polymeric material such as; polypropylene, polyethylene or mixtures thereof.

In the state of the art, a polymeric film is laminated to a nonwoven web to form a backsheet (12) and prevents liquid leakage from the absorbent article (1).

Any known backsheet formation process can be applied in the present invention. The backsheet formed in the present invention has substantially non-elastic nature.

In the present invention, at least one elastomeric strand (14) and/or strip (16) is attached on the interior surface of the polymeric film layer of said backsheet in extended form such that; when in relaxed state, said elastomeric strand (14) and/or strip (16) reduces the length of the absorbent article (1) in some extent.

The elastomeric strand(s) (14) or strip(s) (16) material may be chosen from elastic materials which can comprise elastomeric polymers such as block copolymers comprising SBS and/or SEBS, styrene block copolymers, polyesters, polyurethanes, polyethers and polyether block copolymers, latex, rubber based polymers or combinations thereof. In the present invention polyurethane or rubber based polymers are preferred as elastomeric strand (14) or strip (16) material, more preferably polyurethane is used.

According to the present invention the elastomeric strand(s) (14) and/or strip(s) (16) is attached on the backsheet localized substantially in the range of the absorbent core (10) which prevents sagging of the absorbent article (1). The elastomeric strand(s) (14) and/or strip(s) (16) may be symmetrically oriented in the core width (W) range.

The details of the present invention is given further as following embodiments.

In one embodiment, the backsheet (12) of the present invention is formed by laminating the polymer film layer (18) and NW web layer (20) together. Before or after lamination of these two layers, one or more elastomeric strands (14) and/or strips (16) are attached on polymeric film layer (18). Said polymeric film layer (18) and NW web layer (20) form interior surface and exterior surface of the backsheet (12).

The polymeric film layer (18) forms the interior surface of the backsheet (12) which is directly in contact with the absorbent core (10). Said polymeric film layer (18) has two surfaces; the interior surface is attached to absorbent core (10) of the absorbent article and the exterior surface is attached to the NW web layer (20).

The nonwoven web layer (20) forms the exterior face of the backsheet (12) which is directly in contact with the garment of the wearer. Said NW web layer (20) has two surfaces. The interior surface of said NW web layer (20) is attached to the exterior surface of the polymeric film layer (18) and the exterior surface of said NW web layer (20) is directly in contact with the garment of the user and with the legs of the wearer.

According to the present invention the elastomeric strand(s) (14) is attached on the interior surface of the polymeric film layer (18).

By attachment of the elastomeric strand(s) (14) and/or strip(s) (16) on the exterior surface of the polymeric film layer (18), the shape of the backsheet (12) may seem over crimpled. Furthermore, the elastomeric strip(s) (16) can not be attached robust and as a consequence, the elastomeric strand(s) (14) can be detached from the backsheet surface. Therefore, the elastomeric strand(s) is attached on the interior surface of the polymeric film layer (18) in the present invention.

In one embodiment, the elastomeric strand(s) (14) and/or strip(s) (16) is stretched to some extent before attaching on the backsheet (12), in flat position. Afterwards, they are attached on the backsheet (12), preferably on the polymeric film layer surface (18). The elastomeric strand(s) (14) is extended up to some extend to obtain an absorbent article having crinkled backsheet at the crotch region in relaxed state of the absorbent article.

In one embodiment, the extended form of the elastomeric strand(s) (14) and/or strip(s) (16) is attached in/on polymeric film layer (18) and/or the nonwoven web layer (20) of the backsheet (12) in longitudinal and/or substantially curved longitudinal structure.

In the present invention, the elastomeric strand(s) (14) and/or strip(s) (16) is stretched to an extend as such; enabling up to 50 % stretch in longitudinal direction of the absorbent article (1).

According to the present invention, the elastomeric strand(s) (14) may be extended, exemplarily for polyurethane based elastomeric strands with diameter of around 500 micron, up to 300 % of its original length, preferably 250 %, more preferably 200 % of its original length to provide up to 50 % of stretch in longitudinal direction of the absorbent article (1).

In one embodiment, the extended form of the elastomeric strand(s) (14) and/or strip(s) (16) is attached in/on polymeric film layer (18) of the backsheet (12), preferably attached on polymeric film layer (18).

In another embodiment, the elastomeric strand(s) (14) and/or strip(s) (16) is localized preferably, substantially in the range of absorbent core(10) of the absorbent article (1). Whereas, the length of the elastomeric strand(s) (14) and/or strip(s) (16) may be longer than the absorbent core length in longitudinal axis (X).

According to the present invention, the length of said elastomeric strand(s) (14) and/or strip(s) (16) extends between 5 to 95 %, preferably between 20 to 70 % of the length (L) in longitudinal axis of the absorbent article (1).

In another embodiment, the elastomeric strand (14) and/or strip (16) forms channel like structures on the backsheet (12) by lying under the absorbent core (10) of the absorbent article (1).

The channels can be formed by at least one elastomeric strand (14) and/or at least one elastomeric strip (16). Said elastomeric strip (16) may be formed by at least one strand (14) or by a plurality of elastomeric strands (14). These formed channels are permanent which means that; the integrity of the channels are maintained both in dry state and wet state.

In one embodiment, backsheet (12) may be crinkled when the absorbent article (1) is in use. Said backsheet is formed by attaching at least one stretched elastomeric strand (14) and/or strip (16) when the article is in flat position.

The term bonded or attached, used herein, expresses configurations whereby an element is directly joined to another element by affixing the element directly to the other element, and configurations whereby an element is indirectly joined to another element by affixing the element to intermediate member(s) which in turn are affixed to the other element.

The term "stretched or extended" used herein, refers to the length of the material as increased or extended with regard to the original length.

The term used as "original length" refers to the length of the material in non-stretched state or non crimpled state.

The term elastic or elastomeric polymer refers to a material stretchable up to 300 % or more of its original length under stress, and can return to its original length when the applied stretching force is removed.

The term substantially refers to a large extent.

According to the present invention, the backsheet (12) of the absorbent article (1) comprises one or more elastomeric strand (14) and/or, one or more elastomeric strip (16).

Elastomeric strip(s) (16) consist of one or a plurality of elastomeric strand(s) (14).

In one embodiment, the backsheet(12) comprises at least one elastomeric strip (16) comprising at least one elastomeric strand (14). Said elastomeric strip(s) has a width (W) and a length (L).

According to the present invention, the elastomeric strip (16) has a width (W) that is formed of at least one elastomeric strand (14).

In one embodiment, the backsheet (12) of the absorbent article (1) may comprise one elastomeric strip consists of one elastomeric strand (14) or, at least two elastomeric strips (16) consist of one elastomeric strand (14) respectively.

In another embodiment, the backsheet (12) of the absorbent article (1) may comprise one elastomeric strip (16) consists of at least two elastomeric strands (14) or, at least two elastomeric strips (16) consist of at least two elastomeric strands (14) respectively.

In one embodiment, one or more of elastomeric strand (14) and/or strip (16) bonded on the polymeric film layer (18), which is made from polyurethane and/or its mixture with latex and/or rubber based polymers.

According to the present invention; said elastomeric strand(s) (14) and/or strip(s) (16) is preferably made from polyurethane.

According to the present invention; the diameter of said single polyurethane elastomeric strand (14) is between preferably 200 to 800 micron, more preferably between 400 to 600 micron.

According to the present invention, the distance between two elastomeric strips (16) is at least 5 mm, more preferably 10 mm. Whereas, the distance (B) between two elastomeric strips (16) is measured as; between the outer elastomeric strands (16) of each elastomeric strip (14) that facing each other.

According to the present invention, said at least two strips are located preferably centrally with regard to the width (W) of the absorbent core (10).

According to the present invention; the width (W) of said elastomeric strips (16) comprising at least one elastomeric strand (14) is at most 80 % of the width (W) of the absorbent core (10), preferably at most 40 %, more preferably at most 20 % of the width (W) of the absorbent core (10).

According to the present invention; the channels, formed by elastomeric strands (14) and/or strips (16) on backsheet (12) of the article, are permanent.

"Permanent" used herein means that, the channel structures are maintained in the same structure without deforming the absorbent article (1) in both dry state and wet state.

According to the present invention; elastomeric strand(s) (14) and/or strips (16) are localized substantially under the absorbent core (10) and attached on the backsheet (12), to form channel like structures.

In dry state of the absorbent article (1), formed channels holds the absorbent article (1) and provides comfort for the wearer. Attached elastomeric strand(s) (14) and/or strip(s) (16) crinkles the backsheet (12) structure which helps to fit the article to the body of the wearer.

In wet state of the absorbent article (1), formed channels holds the absorbent article (1) which is filled up with body exudates. Furthermore, it provides fit shape structure on user's buttocks and prevents leakage of the body fluids or feces.

Particularly in the present invention, the channels are resistant to friction forces and movements of the wearer. The channels do not burst by harsh movements of users.

In one embodiment, the permanent channels are obtained by attaching elastomeric strand(s) (14) or strip(s) (16) on interior face of the backsheet (12) as polymeric film layer (18) followed by laminating the polymeric film layer (18) on to the nonwoven web layer (20).

In one embodiment, the polymeric film layer (18) is laminated on to the nonwoven web layer (20), followed by attaching the elastomeric strand(s) (14) and/or strip(s) (16) on the interior surface of the polymeric film layer (18) which is body facing side.

In another embodiment, the elastomeric strand(s) (14) and/or strip(s) (16) is attached on the exterior surface of the polymeric film layer (18) which is garment facing side, followed by lamination to the nonwoven web layer (20).

According to the present invention, one or more of elastomeric strand(s) (14) and/or strip(s) (16) is bonded on the interior side of the polymeric film layer (18).

In one embodiment, the elastomeric strand(s) (14) and/or strip(s) (16) is attached on the polymeric film layer (18), by applying a thermoplastic adhesive material wherein used thermoplastic adhesive is preferably hot melt adhesive.

In one embodiment said hot melt adhesive can be applied by extruding, rolling or spraying on the surface of the elastomeric strand(s) (14) and/or strip(s) (16).

Hot melt adhesive used in the present invention can be chosen from; hydrocarbon, polyolefin, resin or rubber based polymers. Polyolefin based polymers such as; polyethylene, polypropylene, polybutene-1, oxidized polyethylene, polyisoprene or polybutadiene may be in form of block copolymers including A-B-A triblock structures, A-B diblock structures and (A-B)n radial block copolymer structures may be used. Rubber based polymers such as; Styrene block copolymers (SBC), also called styrene copolymer adhesives and rubber-based adhesives which have good low-temperature flexibility, high elongation, and high heat resistance can be used. Said rubber based polymers usually have A-B-A structure, with an elastic rubber segment between two rigid plastic endblocks such as; Styrene-butadiene-styrene (SBS), Styrene-isoprene-styrene (SIS), Styrene-ethylene/butylene-styrene (SEBS) or Styrene-ethylene/propylene (SEP).

According to the present invention; preferred holt melt adhesive is rubber based hot melt adhesive such as styrene-rubber-copolymer.

According to the present invention, an absorbent article is manufactired with a process comprising the steps;
i) Stretching one or more elastomeric strand(s) (14) up to an extend, to provide up to 50 % of stretch in length (L) in longitudinal direction of the absorbent article,
ii) Attaching the stretched elastomeric strand(s) (14) on the interior surface of the polymeric film layer (18) of the backsheet (12) by using hot melt adhesive,
iii) Laying said backsheet (12) having attached elastomeric strands (14), in stretched form in flat position as bottom layer,
iv) Attaching the absorbent core (10) on the backsheet (12),
v) Laying the topsheet (6) on the absorbent core (10) and backsheet (12) to cover the surface and form the absorbent article (1)

The production of the absorbent article (1) having elastomeric strand(s) (14) and/or strip(s) (16) is disclosed in Examples with further details below.

### EXAMPLE 1: Production Of The Absorbent Article (1) Of The Present Invention

The absorbent article (1) of the present invention, having elastomeric strand(s) (14) and/or strip(s) (16), can be manufactured by the following steps;
- Laminating the polymeric film layer (18) and the nonwoven web layer (20) together to form the backsheet (12),
- Stretching one or more elastomeric strand(s) (14) up to 300 % of its original length wherein, stretched form of elastomeric strand(s) (14) enables up to 50 % stretch in length (L) in longitudinal direction of the absorbent article (1) to reach original length of said absorbent article,
- Attaching the stretched elastomeric strand(s) (14) on the interior surface of the polymeric film layer (18) of the backsheet (12) in the absorbent core region by using hot melt adhesive,
- Laying said backsheet (12) with attached elastomeric strands (14), in stretched form in flat position as bottom layer,
- Laying absorbent core (10) on the backsheet (12),
- Laying ADL (8) on the absorbent core (10),
- Laying the topsheet (6) on the ADL (8) and backsheet (12) to cover the surface and form the absorbent article (1).

### Example 2 not according to the invention:

### Production Of The Absorbent Article (1) Of The Present Invention

The absorbent article (1) of the present invention, having elastomeric strand(s) (14) and/or strip(s) (16), can be manufactured by the following steps;
- Stretching one or more elastomeric strand(s) (14) up to 300 % of its original length wherein, stretched form of elastomeric strand(s) (14) enables up to 50 % stretch in length (L) in longitudinal direction of the absorbent article (1) to reach original length of said absorbent article,
- Attaching the stretched elastomeric strand(s) (14) on the exterior surface of the polymeric film layer (18) of the backsheet (12) in the absorbent core region, by using hot melt adhesive,
- Laminating the polymeric film layer (18) and the nonwoven web layer (20) together to form the backsheet (12),
- Laying said backsheet (12) with attached elastomeric strands (14), in stretched form in flat position as bottom layer,
- Laying the absorbent core (10) on the backsheet (12),
- Laying ADL (8) on the absorbent core (10),
- Laying the topsheet (6) on the ADL (8) and backsheet (12) to cover the surface and form the absorbent article (1).

The production of the absorbent article (1) having elastomeric strand(s) (14) and/or strip(s) (16) in the present invention is further disclosed in the following Example 3. The example is given for illustrative purposes and should not limit the scope of the invention.

### EXAMPLE 3: Production Of The Absorbent Article (1) Of The Present Invention

The absorbent article (1) of the present invention, having elastomeric strand(s) (14) and/or strip(s) (16), can be manufactured by the following steps;
- Laminating the polymeric film layer (18) made from polyethylene based polymer having a thickness of around 15 micron and the nonwoven web layer (20) together to form the backsheet (12) having a length of 485 mm,
- Stretching two elastomeric strips (16) having two elastomeric strands (14) each, up to 200 % of its original length and hot melt adhesive is applied on to said elastomeric strand(s) (14), wherein each elastomeric strand is made form polyurethane based polymer having a diameter of 500 micron,
- Attaching the stretched elastomeric strand(s) (14) on the interior surface of the polymeric film layer (18) of the backsheet (12) centrally located in the absorbent core region wherein, the distance between two strands is 3 mm and the distance between two strips is 30 mm by a width of absorbent core of 90 mm,
- Laying said backsheet (12) with attached elastomeric strands (14), in stretched form in flat position as bottom layer,
- Laying the absorbent core (10) on the backsheet (12),
- Laying ADL (8) on the absorbent core (10),
- Laying the topsheet (6) on the ADL (8) and backsheet (12) to cover the surface and form the absorbent article (1),
- Releasing the applied stretch on the absorbent article to obtain a crimpled structure of said absorbent article,
- In this way obtained absorbent article has a length of 390 mm,
- Accordingly, a 20 % reduction of the length of the absorbent article is achieved,

Whereas, a test method for measurement of the reduced length is applied that is specified in the present application below.

According to the present invention, the adhesives may be used for impregnation of elastomeric strand (14) on the polymeric film layer (18).

Using adhesive for bonding of elastomeric strand (14) on the polymeric film layer (18) is a preferred method in the present invention. But also, other known methods, for example ultrasonic or heat bonding may be applied.

The present invention provides an absorbent article (1) for personal hygiene as indicated in the claims. The absorbent article (1) has a front edge and a back edge, a longitudinal axis extending in longitudinal direction and a lateral axis extending in lateral direction of the absorbent article.

In one embodiment, the elastomeric strand(s) (14) and/or strip(s) (16) may be bonded on backsheet (12) in longitudinal axis (X) or in substantially curved form in the longitudinal axis(X), when the absorbent article (1) is in flat position. The preferred attachment direction is in longitudinal (X) axis.

In the present invention, it is preferred to attach the elastomeric strand(s) (14) and/or strip(s) (16) on backsheet (12) in longitudinal axis (X), which provides channel like structure on absorbent core (10) and best fit shape to the user as shown in Figs 1-8.

In the present invention, the elastomeric strand(s) (14) and/or strip(s) (16) can be attached on the backsheet (12) in substantially curved longitudinal axis (X), which can also provide channel like structure on absorbent core (10) and best fit shape to the user.

In the present invention, when in relaxed state, said elastomeric strand(s) (14) and/or strip(s) (16) on backsheet (12) in longitudinal axis (X) reduces the length of said absorbent article (1) by crinkling the backsheet structure as shown in Fig. 9b.

In the present invention, said elastomeric strip(s) (16) is attached on/in said polymeric film layer (18) of the backsheet (12) in longitudinal axis (X) in stretched form to provide up to 50 % stretch in longitudinal direction of the absorbent article (1) to reach original length of said absorbent article (1) by stretching.

### Test Method for Measuring The Reduced Length Of The Absorbent Article:

The reduced length of the absorbent article is measured according to the method comprising the following steps;
- The leg cuffs of the reference absorbent article without elastomeric strand(s) (14) and/or strip(s) (16), are cut and removed from the absorbent article,
- The reference absorbent article, is then put on a plane surface in flat position,
- The length of the reference absorbent article is measured by using a ruler,
- Providing an absorbent article of the present invention with elastomeric strand(s) (14) and/or strip(s) (16) having a backsheet, having same original length with the reference absorbent article,
- The leg cuffs of the absorbent article of the present invention, are cut and removed from the absorbent article,
- The remaining absorbent article of the present invention is put on a plane surface in flat position,
- The length of the absorbent article of the present invention is measured by using a ruler and,
- The length difference between the reference absorbent article and the absorbent article of the present invention is calculated.

According to present above described in-house test method, the reduced amount of the length of the absorbent article (1) is calculated. Stretching elastomeric strand(s) (14), reduces the length of the absorbent article in some extent.

According to the present invention the stretching amount of the elastomeric strand(s) enables, up to 50 % stretch in longitudinal direction of the absorbent article (1) to reach original length of said absorbent article (1) according to the given in-house test method.

In the present invention, the backsheet (12) of the absorbent article (1) comprises elastomeric strand(s) (14) and/or strip(s) (16) in its longitudinal axis (X). The length (L) of the elastomeric strand(s) and/or strip(s) comprise at least 5 % of the longitudinal length of the absorbent article(1), and at most 95 % of the longitudinal length of the absorbent article(1).

In the present invention, the elastomeric strand(s) (14) and/or strip(s) (16) that attached on the backsheet (12) forms at least two channels which are at least partially oriented in the longitudinal direction of the absorbent article.

The lenghth of said elastomeric strand(s) (14) and/or strip(s) (16) lying in the longitudinal axis of the absorbent article (1) is between 5 to 95 % of the longitudinal length (L) of the absorbent article (1), preferably between 20 to 70 % of the longitudinal length of the absorbent article.

**In** the present invention, the preferred maximum length of the elastomeric strand(s) (14) and/or strip(s) (16) lying in the longitudinal axis is at most 70 % of the longitudinal length of the absorbent article, since the backsheet loose its crinkled shape when the length of the elastomeric strand (14) and/or strip is more than 70 % of the longitudinal length of the absorbent article.

**In** the present invention, the preferred minimum length of the elastomeric strand(s) (14) and/or strip(s) (16) lying in the longitudinal axis is at least 20 % of the longitudinal length of the absorbent article, since it is not effective to prevent sagging problem below said value.

## Claims

1. A pant or diaper (1) having longitudinal axis (X) and lateral axis (Y) comprising, a backsheet (12), a topsheet (6) and an absorbent core (10) layered between the topsheet (6) and the backsheet (12), wherein said backsheet (12) comprises;
i) a polymeric film layer (18) as the interior face of the backsheet (12) and,
ii) a nonwoven web layer (20) as the exterior face of the backsheet (12),
wherein;
said polymeric film layer (18) having;
- an interior surface, that is in contact with the absorbent core (10) and an exterior surface, that is in contact with the nonwoven web layer (20) and,
said nonwoven web layer (20) having;
- an interior surface, that is in contact with said polymeric film layer (18) and an exterior surface, that is in contact with the garment of the wearer,
**characterized in that**;
said backsheet comprises; at least one elastomeric strip (16) having a width (W) and a length (L) comprising at least one elastomeric strand (14)
wherein,
said elastomeric strip(s) (16) is localized substantially in the range of absorbent core (10) and the distance between two elastomeric strips (16) (B), is at least 5 mm, preferably at least 10 mm,
wherein,
said elastomeric strip(s) (16) is attached on the interior surface of the polymeric film layer (18) of said backsheet (12) in longitudinal axis (X), in stretched form such that; when in relaxed state, said elastomeric strip(s) (16) reduces the length of said pant or diaper (1).

2. A pant or diaper (1) according to claim 1, wherein, said elastomeric strip(s) (16) is attached on/in said polymeric film layer (18) of the backsheet (12) in longitudinal axis (X) in stretched form enabling up to 50 % stretch in longitudinal direction of the length (L) of said pant or diaper (1).

3. A pant or diaper (1) comprising elastomeric strip(s) (16) according to claim 1, wherein the length of said elastomeric strip(s) (16) extends between 5 to 95 %, preferably between 20 to 70 % of the length (L) of the pant or diaper (1).

4. A pant or diaper (1) comprising elastomeric strip(s) (16) having a width (W) according to claim 1, wherein the width (W) of said elastomeric strip is at most 80 % of the width (W) of the absorbent core (10), preferably at most 40 %, more preferably at most 20 % of the width (W) of the absorbent core (10).

5. A pant or diaper (1) comprising elastomeric strand (14) according to claim 1, wherein said elastomeric strand (14) is formed of an elastic material comprising styrene block copolymers, polyesters, polyurethanes, polyethers and polyether block copolymers, latex, rubber based polymers or mixtures thereof, preferably polyurethane.

6. A pant or diaper (1) comprising elastomeric strand(s) (14) formed of polyurethane according to claim 5, wherein the diameter of said polyurethane elastomeric strand (14) is between 200 to 800 micron, preferably between 400 to 600 micron.

7. A pant or diaper (1) comprising elastomeric strand(s) (14) according to claim 1, wherein said elastomeric strand(s) (16) is attached on the polymeric film layer (18) of the backsheet (12) with a hot melt adhesive.

8. A pant or diaper (1) comprising elastomeric strip(s) (16) comprising elastomeric strand(s) (14) according to claim 1, wherein polymeric film layer (18) of the backsheet (12) comprises preferably two elastomeric strips (16) located centrally in the absorbent core width range, comprising at least one elastomeric strand (14).

9. A process of manufacturing of a pant or diaper (1) according to claim 1, comprising the steps;
i) Stretching one or more elastomeric strand(s) (14) in an amount, to provide up to 50 % of stretch in longitudinal direction of the length (L) of the pant or diaper (1).
ii) attaching the stretched elastomeric strand(s) (14) on the interior surface of the polymeric film layer (18) of the backsheet (12) by using hot melt adhesive,
iii) laying said backsheet (12) with attached elastomeric strands (14), in stretched form in flat position as bottom layer,
iv) attaching absorbent core (10) on the backsheet (12),
v) laying topsheet (6) on the absorbent core (10) and backsheet (12) to cover the surface and form the pant or diaper (1).

10. A pant or diaper (1) according to any of claims 1-8 wherein, said pant or diaper (1) is used as disposable or wearable article comprising baby diapers, incontinence diapers, pants or pull ups.

## Patentansprüche

1. Windelhose oder Windel (1) mit einer Längsachse (X) und einer Querachse (Y), umfassend eine Rückseitenlage (12), eine Oberlagenlage (6) sowie einen Saugkern (10), der zwischen der Oberlagenlage (6) und der Rückseitenlage (12) angeordnet ist,
**dadurch gekennzeichnet, daß** die Rückseitenlage (12) folgendes aufweist:
i) eine polymere Folienlage (18) als innere Fläche der Rückseitenlage (12), und
ii) eine Vliesstofflage (20) als äußere Fläche der Rückseitenlage (12),
wobei
die polymere Folienlage (18)
- eine Innenfläche aufweist, die mit dem Saugkern (10) in Kontakt steht, sowie eine Außenfläche, die mit der Vliesstofflage (20) in Kontakt steht, und
die Vliesstofflage (20)
- eine Innenfläche aufweist, die mit der polymeren Folienlage (18) in Kontakt steht, sowie eine Außenfläche, die mit dem Kleidungsstück des Trägers in Kontakt steht,
**dadurch gekennzeichnet, daß**
die Rückseitenlage (12) mindestens einen elastomeren Streifen (16) mit einer Breite (W) und einer Länge (L) enthält, welcher mindestens einen elastomeren Faden (14) umfaßt,
wobei
der oder die elastomeren Streifen (16) im wesentlichen im Bereich des Saugkerns (10) lokalisiert sind und der Abstand (B) zwischen zwei elastomeren Streifen (16) mindestens 5 mm, vorzugsweise mindestens 10 mm beträgt,
wobei ferner
der oder die elastomeren Streifen (16) auf der Innenfläche der polymeren Folienlage (18) der Rückseitenlage (12) entlang der Längsachse (X) in gedehntem Zustand befestigt sind, so daß sie im entspannten Zustand die Länge der Windelhose oder Windel (1) verkürzen.

2. Windelhose oder Windel (1) nach Anspruch 1, **dadurch gekennzeichnet, daß** der oder die elastomeren Streifen (16) auf bzw. in der polymeren Folienlage (18) der Rückseitenlage (12) entlang der Längsachse (X) in gedehntem Zustand befestigt sind, wodurch eine Dehnung von bis zu 50 % in Längsrichtung der Länge (L) der Windelhose oder Windel (1) ermöglicht wird.

3. Windelhose oder Windel (1) mit elastomeren Streifen (16) gemäß Anspruch 1, wobei die Länge der elastomeren Streifen (16) zwischen 5 % und 95 %, vorzugsweise zwischen 20 % und 70 % der Länge (L) der Windelhose oder Windel (1) beträgt.

4. Windelhose oder Windel (1) mit elastomeren Streifen (16) mit einer Breite (W) gemäß Anspruch 1, wobei die Breite (W) des elastomeren Streifens höchstens 80 %, vorzugsweise höchstens 40 %, besonders bevorzugt höchstens 20 % der Breite (W) des Saugkerns (10) beträgt.

5. Windelhose oder Windel (1) mit einem elastomeren Faden (14) gemäß Anspruch 1, wobei der elastomere Faden (14) aus einem elastischen Material besteht, das Styrol-Blockcopolymere, Polyester, Polyurethane, Polyether und Polyether-Blockcopolymere, Latex, kautschukbasierte Polymere oder deren Mischungen umfaßt, vorzugsweise Polyurethan.

6. Windelhose oder Windel (1) mit einem aus Polyurethan bestehenden elastomeren Faden (14) gemäß Anspruch 5, wobei der Durchmesser des Polyurethan-Elastomerfadens (14) zwischen 200 und 800 Mikrometer, vorzugsweise zwischen 400 und 600 Mikrometer liegt.

7. Windelhose oder Windel (1) mit einem oder mehreren elastomeren Fäden (14) gemäß Anspruch 1, wobei der oder die elastomeren Fäden (14) mit einem Heißschmelzklebstoff auf der polymeren Folienlage (18) der Rückseitenlage (12) befestigt sind.

8. Windelhose oder Windel (1) mit einem oder mehreren elastomeren Streifen (16), die jeweils mindestens einen elastomeren Faden (14) gemäß Anspruch 1 enthalten, wobei die polymere Folienlage (18) der Rückseitenlage (12) vorzugsweise zwei elastomere Streifen (16) umfaßt, die zentral im Breitenbereich des Saugkerns angeordnet sind und jeweils mindestens einen elastomeren Faden (14) enthalten.

9. Verfahren zur Herstellung einer Windelhose oder Windel (1) gemäß Anspruch 1, umfassend die folgenden Schritte:
**i)** Dehnen eines oder mehrerer elastomerer Fäden (14) in einem Ausmaß, das eine Dehnung von bis zu 50 % in Längsrichtung der Länge (L) der Windelhose oder Windel (1) ermöglicht,
**ii)** Befestigen der gedehnten elastomeren Fäden (14) auf der Innenfläche der polymeren Folienlage (18) der Rückseitenlage (12) unter Verwendung eines Heißschmelzklebstoffs,
**iii)** Ablegen der Rückseitenlage (12) mit den befestigten elastomeren Fäden (14) in gedehntem Zustand in flacher Position als untere Lage,
**iv)** Befestigen des Saugkerns (10) auf der Rückseitenlage (12),
**v)** Ablegen der Oberlagenlage (6) auf dem Saugkern (10) und der Rückseitenlage (12), um die Oberfläche zu bedecken und die Windelhose oder Windel (1) zu bilden.

10. Windelhose oder Windel (1) nach einem der Ansprüche 1 bis 8, wobei die Windelhose oder Windel (1) als wegwerfbarer oder tragbarer Artikel verwendet wird, der Babywindeln, Inkontinenzwindeln, Windelhosen oder sogenannte Pull-up-Hosen umfaßt.

## Revendications

1. Couche-culotte ou couche (1) ayant un axe longitudinal (X) et un axe transversal (Y), comprenant une feuille arrière (12), une feuille supérieure (6) et un noyau absorbant (10) disposé entre la feuille supérieure (6) et la feuille arrière (12),
dans laquelle ladite feuille arrière (12) comprend :
i) une couche de film polymère (18) constituant la face interne de la feuille arrière (12), et
ii) une couche de voile non tissé (nonwoven) (20) constituant la face externe de la feuille arrière (12),
dans laquelle :
la couche de film polymère (18) présente :
- une surface interne en contact avec le noyau absorbant (10) et une surface externe en contact avec la couche de voile non tissé (20), et
la couche de voile non tissé (20) présente :
- une surface interne en contact avec ladite couche de film polymère (18) et une surface externe en contact avec le vêtement de l'utilisateur,
**caractérisée en ce que**
ladite feuille arrière (12) comprend au moins une bande élastomère (16) ayant une largeur (W) et une longueur (L) et comprenant au moins un fil élastomère (14),
dans laquelle :
ladite ou lesdites bandes élastomères (16) sont localisées sensiblement dans la zone du noyau absorbant (10), et la distance (B) entre deux bandes élastomères (16) est d'au moins 5 mm, de préférence d'au moins 10 mm,
dans laquelle :
ladite ou lesdites bandes élastomères (16) sont fixées sur la surface interne de la couche de film polymère (18) de ladite feuille arrière (12), le long de l'axe longitudinal (X), à l'état étiré, de sorte que, à l'état détendu, ladite ou lesdites bandes élastomères (16) réduisent la longueur de la couche-culotte ou de la couche (1).

2. Couche-culotte ou couche (1) selon la revendication 1, dans laquelle ladite ou lesdites bandes élastomères (16) sont fixées sur ou dans la couche de film polymère (18) de la feuille arrière (12), le long de l'axe longitudinal (X), à l'état étiré, permettant une extension pouvant atteindre jusqu'à 50 % dans la direction longitudinale de la longueur (L) de la couche-culotte ou de la couche (1).

3. Couche-culotte ou couche (1) comprenant une ou plusieurs bandes élastomères (16) selon la revendication 1, dans laquelle la longueur desdites bandes élastomères (16) s'étend entre 5 % et 95 %, de préférence entre 20 % et 70 % de la longueur (L) de la couche-culotte ou de la couche (1).

4. Couche-culotte ou couche (1) comprenant une ou plusieurs bandes élastomères (16) ayant une largeur (W) selon la revendication 1, dans laquelle la largeur (W) de ladite bande élastomère représente au maximum 80 % de la largeur (W) du noyau absorbant (10), de préférence au maximum 40 %, et plus préférentiellement au maximum 20 % de la largeur (W) du noyau absorbant (10).

5. Couche-culotte ou couche (1) comprenant au moins un fil élastomère (14) selon la revendication 1, dans laquelle ledit fil élastomère (14) est constitué d'un matériau élastique comprenant des copolymères en bloc de styrène, des polyesters, des polyuréthanes, des polyéthers et des copolymères en bloc de polyéther, du latex, des polymères à base de caoutchouc ou leurs mélanges, de préférence du polyuréthane.

6. Couche-culotte ou couche (1) comprenant un ou plusieurs fils élastomères (14) constitués de polyuréthane selon la revendication 5, dans laquelle le diamètre dudit fil élastomère (14) en polyuréthane est compris entre 200 et 800 microns, de préférence entre 400 et 600 microns.

7. Couche-culotte ou couche (1) comprenant un ou plusieurs fils élastomères (14) selon la revendication 1, dans laquelle le ou lesdits fils élastomères (14) sont fixés sur la couche de film polymère (18) de la feuille arrière (12) au moyen d'un adhésif thermofusible.

8. Couche-culotte ou couche (1) comprenant une ou plusieurs bandes élastomères (16) comportant au moins un fil élastomère (14) selon la revendication 1, dans laquelle la couche de film polymère (18) de la feuille arrière (12) comprend de préférence deux bandes élastomères (16) situées au centre de la plage de largeur du noyau absorbant et comportant chacune au moins un fil élastomère (14).

9. Procédé de fabrication d'une couche-culotte ou d'une couche (1) selon la revendication 1, comprenant les étapes suivantes :
i) étirer un ou plusieurs fils élastomères (14) dans une mesure permettant une extension pouvant atteindre 50 % dans la direction longitudinale de la longueur (L) de la couche-culotte ou de la couche (1) ;
ii) fixer les fils élastomères (14) ainsi étirés sur la surface interne de la couche de film polymère (18) de la feuille arrière (12) à l'aide d'un adhésif thermofusible ;
iii) disposer ladite feuille arrière (12) portant les fils élastomères (14) fixés, à l'état étiré, à plat en position de couche inférieure ;
iv) fixer le noyau absorbant (10) sur la feuille arrière (12) ;
v) disposer la feuille supérieure (6) sur le noyau absorbant (10) et la feuille arrière (12) de manière à recouvrir la surface et à former la couche-culotte ou la couche (1).

10. Couche-culotte ou couche (1) selon l'une quelconque des revendications 1 à 8, dans laquelle la couche-culotte ou la couche (1) est utilisée comme article jetable ou portable, comprenant des couches pour bébés, des couches pour l'incontinence, des culottes absorbantes ou des couches-culottes d'apprentissage (pull-ups).
